# EUROPEAN PATENT SPECIFICATION

(11) **EP 3 846 689 B1**
(45) Date of publication and mention of the grant of the patent: **11.06.2025**
(21) Application number: 19774181.2
(22) Date of filing: 03.09.2019
(51) Int. Cl.: A61B 5/053

(54) **IMPEDENTIOMETRIC SYSTEM FOR THE ASSESSMENT OF MUSCLE MASS**
IMPEDENTIOMETRISCHES SYSTEM ZUR BEWERTUNG DER MUSKELMASSE
SYSTÈME D'IMPÉDANCE POUR L'ÉVALUATION DE LA MASSE MUSCULAIRE

(30) Priority: 10.09.2018 IT 201800008470
(43) Date of publication of application: 14.07.2021
(73) Proprietor: Alma Mater Studiorum Universita di Bologna, 40126 Bologna (IT)
(72) Inventor: DOMENICALI, Marco, 40126 Bologna (IT); LENZI, Enrico, 40126 Bologna (IT); PICCIOLI, Riccardo, 40126 Bologna (IT); VIROLI, Daniela, 40126 Bologna (IT)
(74) Representative: Minghetti, Mauro
(86) International application number: PCT/IB2019/057418
(87) International publication number: WO 2020/053703

(56) References cited:
- WO-A1-2011/022068
- JP-A- S63 222 241
- US-A1- 2004 059 242
- US-A1- 2015 272 501

## Description

### TECHNICAL FIELD OF THE INVENTION

The present invention relates to an impedentiometric system for the assessment of muscle mass.

In more detail, the present invention relates to a new-generation impedentiometric system for the measurement of muscle mass in the elderly.

### DESCRIPTION OF THE PRIOR ART

Currently, the most accurate analysis methodologies for the assessment of bodily composition, and in particular of the amount of muscle mass, are imaging techniques such as computed tomography, magnetic resonance, and Dual energy X-ray Absorptiometry (DXA).

Computed tomography measures, with high accuracy, the areas of the cross-sections and the volume of muscles; it also allows assessing muscle density (parameter correlated to the infiltration of fat mass in muscles) and intramuscular and subcutaneous adipose deposits.

Magnetic resonance is a diagnostic technique which, by means of the generation of images, allows carrying out a three-dimensional evaluation of soft tissues within the human body.

Among the imaging techniques, DXA is the most widely used for its relatively lower cost and availability.

Even if it is not considered a "gold standard" technique, DXA can provide an accurate estimate of the lean, fat and bone components of body tissues, of the body as a whole or divided into segments.

DXA has been shown to be adequate in the senile population (Baumgarten et al., 1995), and was used for one of the first definitions of sarcopenia (Baumgarten et al., 1998).

Furthermore, DXA is one of the techniques for the assessment of body composition recommended by the guidelines of the European Working Group on Sarcopenia in Older People (EWGSOP) (Cruz-Jentoft et al. 2010).

Regardless of how accurate they are, the imaging techniques expose the subject to a non-negligible amount of nuclear radiation (Cesari et al., 2012). Moreover, the high cost of use and transport difficulties limit the possibilities and frequency of use, and make them impractical in situations such as epidemiological studies on large populations, in clinical routines and in large screening programs (Chien et al., 2008).

On the other hand, impedance analysis has the advantage of being simple, inexpensive, non-invasive and easily repeatable, so as to be able to monitor the patient's response to the proposed therapies.

The traditional impedance analysis method (Bioelectrical Impedance Analysis, BIA) allows estimating the body fat mass and the lean mass (National Institutes of Health Technology Assessment Conference Statement or NIH, 1996).

Easy to use with reduced costs, impedance analysis is based on the application of an alternating electric current of low intensity (NIH, 1996) to determine two body impedance components defined resistance (R) and reactance (Xc).

The physiological bases which constitute the rationale for measuring the impedance are based on the observation that the resistance (per unit volume) is negatively correlated with the amount of bodily fluids (body water and lean mass) through which the electric current passes, while the reactance is positively correlated to body cell mass, thanks to the bioelectrical conductivity of cell membranes (Hoffer et al., 1969; Lukaski et al., 1985; Lukaski et al., 1986).

Therefore, from the data acquired related to resistance and reactance, thanks to mathematical models widely described in the scientific literature, an estimation of total body water and lean mass is obtained, and more specifically of muscle mass (NIH, 1996).

The European Working Group on Sarcopenia in Older People (EWGSOP) indicates bioelectrical impedance analysis as a good portable alternative to diagnostic imaging techniques in body composition assessments (Cruz-Jentoft et al., 2010). BIA is also suggested as a technique for systematic and repeated FFM evaluations in clinical practice (Thibault and Pichard., 2012).

Although advantageous from some points of view, impedance analysis does however have limits related to the fact that the mathematical models used can provide, in some situations, information which deviates from reality.

In fact, such information may be significantly influenced by age, sex, ethnicity, state of health of the subject and by the validation method used for the model's development.

Moreover, one of the major limitations in the clinical use of bioelectrical impedance analysis relates to the reproducibility of measurements.

Particularly, but not exclusively, this limitation has greater influence in elderly subjects.

The reproducibility of measurements is primarily influenced by factors related to the instrumentation, related to the subject to be measured, and finally related to the environment.

One of the factors that affects the instrumentation is intra-instrumental variability. The latter consists in an assessment of the reliability of the measurement provided by the same instrument.

To determine this variability, repeated measurements on calibration circuits are carried out, whose impedance value is known.

The periodic verification of this variability is essential in order to be sure that the variations of the measurements are due to changes in the patient's conditions and not to instrumental variations.

Another of the factors that affects the instrumentation is inter-instrumental variability.

The inter-instrumental variability consists in the different impedance value that is obtained when impedance instruments produced by different manufacturers are used in measurements on the same individual.

Differences in the voltage or frequency of the current administered can be at the origin of this discrepancy (Deurenberg et al. 1989), but a high impedance of the electrode-to-skin contact can also be responsible for this phenomenon (Smye et al., 1993).

The problem also arises for impedance instruments produced by the same manufacturer, especially when the measurements are made with different, non-calibrated electrodes.

Therefore, when a study is carried out that requires the use of several impedance instruments, it is essential to evaluate the inter-instrumental variability with appropriate standard validation circuits, which also involve the application of electrodes (as in the previous factor).

Other factors that affect the instrumentation are the electrodes and cables for connection to the instrument, and their characteristics.

The electrodes are of fundamental importance for the purposes of precise measurements.

The electrodes must be of good quality, wholly used and not cut, as is usually done for savings.

Moreover, the electrodes should be strictly disposable, otherwise the lipid film removed from the skin during the measurement of a given individual alters subsequent measurements.

The connection cables, suitably isolated, must run in a rectilinear manner and without coming into contact with each other on a non-conductive surface, away from electromagnetic fields.

Since the computer (or other device), possibly connected to the instrument, is also a source of electromagnetic fields, it should be kept duly away from the connection cables.

In fact, the possibility of any interference increases with the frequency of the current administered.

As for the factors relating to the subject to be measured, the posture assumed by the subject is fundamental for the interpretation of the impedance data.

Normally, the subject lies supine on a flat, non-conductive surface.

The limbs should be spread 30-45° to avoid short circuits produced by the contact between the lower limbs, or between the upper limbs and the trunk (Lukaski et al., 1985).

The passage from the upright position to the supine one involves a rapid reduction of the impedance (3%), due to the movement of liquids from the interstitial slope to the vascular one (Roos et al., 1992).

In addition to the aforesaid rapid variation of the impedance, an intermediate and late variation are distinguished: these have less practical importance because they occur from 40 to 80 and from 80 to 180 minutes respectively from the rapid variation (Kushner et al., 1994).

The postural variations of the impedance are greater at 10 kHz frequencies, where they are deemed to be expressive of the modifications within the extra-cellular liquids; measuring the bioelectrical impedance within 5-10 minutes from the assumption of the supine position is recommended (Deuremberg, 1994).

Another of the factors related to the subject to be measured is the preparation of the skin.

The electrical conductivity of the skin is improved by pretreatment with ethyl or isopropyl alcohol (Kushner, 1992).

This explains the influence of the skin's preparation on an impedance measurement; the pretreatment removes secretions and exfoliated cells from the skin layer.

However, the use of substances containing electrolytes is to be avoided, as they can modify the electrical conductivity of the skin; a further expedient consists in the removal of abundant down from the skin.

Other factors that depend on the subject to be measured are the food and drink consumed by the same.

In fact, the contents of the alimentary canal can interfere with the measurement of the impedance.

In the post-absorption phase, the passage of liquids into the bloodstream can produce spurious values of the impedance: Kushner (1992) therefore recommends that the subject fasts from liquids and solids for at least 2 to 5 hours.

However, other researchers have observed that the error produced by measuring the impedance 2-5 hours from a meal can be acceptable at group level but not in the single individual, therefore they propose 8 hours fasting before the measurement, which is the standard procedure for bioelectrical impedance analysis (BIA).

The respiratory dynamic is also influential on the impedance data, as modifications of the excursion of the thoracic cage can lead to a variation of the conductive volume and, consequently, the impedance itself (Heitmann, 1994).

The disturbances resulting from breathing are around 3 Hz.

Moderate physical exercise is not able to influence bioelectrical impedance analysis (Deutenberg et al., 1988); on the contrary, prolonged physical activity produces artificially low impedance values.

The increase in skin temperature and, possibly, a loss of water prevailing over a loss of electrolytes can explain this phenomenon.

Artefacts of movement linked to the presence of tremors can reduce the reproducibility of the measurement.

Moreover, artefacts caused by the presence of diseases that are associated with the limits listed above, in particular all the diseases that cause sodium retention (such as congestive heart failure, cirrhosis of the liver and renal failure) can be associated with alterations of the reading of the impedance, secondary to the hyper-hydration typical of this situation.

Finally, the environmental factor that can theoretically influence the impedance measurement is the environmental temperature, due to its effects on cutaneous microcirculation and on hydroelectric homoeostasis.

In fact, an average increase of the resistance (R) has been observed (Caton et al., 1988) equal to 8% as a result of lowering the temperature from 35°C to 14°C. Moreover, Garby et al. (1990) observed a decrease of the resistance (R) equal to 2% after 20 minutes of exposure to a temperature of 34°C, as well as an increase of approximately 4% for a subsequent exposure to a temperature of 24°C for 20 minutes; in general, particular attention to the environmental temperature is not required within the range of values comprised between 24°C and 34°C.

All the disruptive factors described above, especially if they occur simultaneously, but also if considered individually, negatively influence the reproducibility of measurements, thus sometimes providing scarcely reliable results.

In the particular case of an elderly subject, factors such as tremors or the frequent change of posture, just to name a few, can determine the onset of interferences or errors of a non-negligible entity.

In order to overcome these problems, numerous specific mathematical models for the characteristics of a given subject have been developed recently, for example age-specific and/or for various ethnic groups; but these specific models can also lead to substantial estimation errors mainly due to the variable level of hydration of the body between individuals, or connected to variations of the state of hydration in the individual patient, or linked to other factors.

Moreover, the development of various mathematical models, in continuous evolution, has created a certain confusion as each instrument currently present on the market presents a limited set of calculation models (often only one single calculation method, in some cases not even indicated by the manufacturer); this limits the comparability of the measurements obtained, even on the same patient with different devices.

US 2015/0272501 discloses a system, method and device for biometric analysis using a wearable device.

The device can be removeably secured to a user's skin surface and it can include a plurality of electrodes positioned to acquire electrical signals from the skin surface; the device can also include an electromyography acquisition module and a skin impedance acquisition module.

The device also includes a processing unit that can use the electrodes to capture a plurality of electrical signals including at least one electromyography signal; the processing unit can generate calibration data based on a subset of the captured electrical signals, and can process the electromyography signals, using the calibration data, to determine a biometric for the user.

US 2004/0059242 discloses an impedentiometric system for the assessment of muscle mass, comprising at least one wearable device, provided with external electrodes to be applied at certain positions on the patient's body, and with an electronic detection part suitable for acquiring the parameters necessary for carrying out the impedance measurements on the subject concerned.

The system also includes a computing platform, configured so as to receive and process the measurement parameters acquired by the electronic detection part, and an interface configured to allow the transmission of impedance measurement data from the wearable device to the cloud platform.

WO 2011/022068 discloses a hand-held device for electrical impedance myography, including electrodes, which may be applied to a region of tissue, and a circuit that measures and generates electrical signals.

The circuit may analyze the measured signal and determine a characteristic of the region of tissue based on the measured signal; the circuit may include a lock-in amplifier for impedance measurement, a computer for performing calculations and a display for displaying the results.

### OBJECTS OF THE INVENTION

The scope of the present invention is to improve the state of the art in the field of bioelectrical impedance analysis.

Within such technical scope, an object of the present invention is to provide an impedentiometric system for the assessment of muscle mass that makes it possible to overcome the drawbacks described above.

Another object of the present invention is to provide an improved impedentiometric system for the assessment of muscle mass as regards the reproducibility of the measurements.

A further object of the present invention is to provide an impedentiometric system for the assessment of muscle mass which is particularly but not exclusively suitable for use on elderly subjects.

Another object of the present invention is to realise an impedentiometric system for the assessment of muscle mass which allows performing the measurements even with the patient in movement (for example during the execution of physical exercise).

Another object of the present invention is to provide an impedentiometric system for the assessment of muscle mass of simple and practical use, and low cost. Another object of the present invention is to devise an impedentiometric system for the assessment of muscle mass which is not dependent on the specific devices and computer equipment used for the processing of the data.

This scope and these objects are achieved by the impedentiometric system for the assessment of muscle mass according to the annexed claim 1.

The impedentiometric system according to the present invention comprises at least one wearable device, provided with external electrodes to be applied at certain positions on the patient's body, and an electronic detection part suitable only to acquire the parameters necessary for carrying out the impedance measurement on the subject concerned.

Furthermore, the system comprises at least one cloud platform, configured so as to receive and process the measurement parameters acquired by said electronic detection part of said wearable device.

The system also comprises at least one interface, configured to allow the transmission of the impedance measurement data from the wearable device to the cloud platform.

The characteristics listed above allow obtaining many advantageous effects, clarified in the description that follows.

Particularly, but not exclusively, the provision of a wearable device allows performing measurements also with the patient in movement, or after the execution of physical exercise, even on a subject (such as an elderly patient) that may have motor disorders or tremors.

Furthermore, the provision of a remote cloud platform interfaced with the wearable device allows performing all the processing and reporting in a manner completely independent from the wearable device itself, which can thus be very light and simple from the functional and constructive point of view.

At the same time, it is possible to carry out various processing on the measurements acquired on the cloud platform, also processed *ad hoc* and suitable to eliminate the influence of disruptive factors (such as, for example, tremors or motor disorders but also others).

According to the invention, the interface between the wearable device and the cloud platform comprises at least one HID interface processor, comprised in the electronic detection part of the wearable device.

This processor is configured to be recognised by the USB system of a computer or other device which can be connected to the internet, such as an HID (Human Interface Device) peripheral device.

This allows transferring the data acquired by the wearable device to the cloud interface in a manner completely independent from the type of device used to perform the transfer, or from the operating system or software installed therein. Dependent claims refer to preferred and advantageous embodiments of the invention.

### BRIEF DESCRIPTION OF DRAWINGS.

The features of the invention will be better understood by anyone skilled in the art from the following description and accompanying drawings, provided by way of non-limiting example, in which:
figure 1 is a schematic perspective view of the impedentiometric system according to the invention and according to a first configuration of the interface between the wearable device and the cloud platform;
figure 2 is another schematic perspective view of the impedentiometric system according to the invention and according to a second configuration of the interface between the wearable device and the cloud platform;
figure 3 is a perspective view of the wearable device of the impedentiometric system according to the invention;
figure 4 is another perspective view, from different angles, of the same device;
figure 5 is a block diagram of the electronic detection part of the device;
figure 6 is a block diagram illustrating the verification procedure of the consistency of the measurements made by the wearable device and sent to the cloud platform.

### EMBODIMENTS OF THE INVENTION.

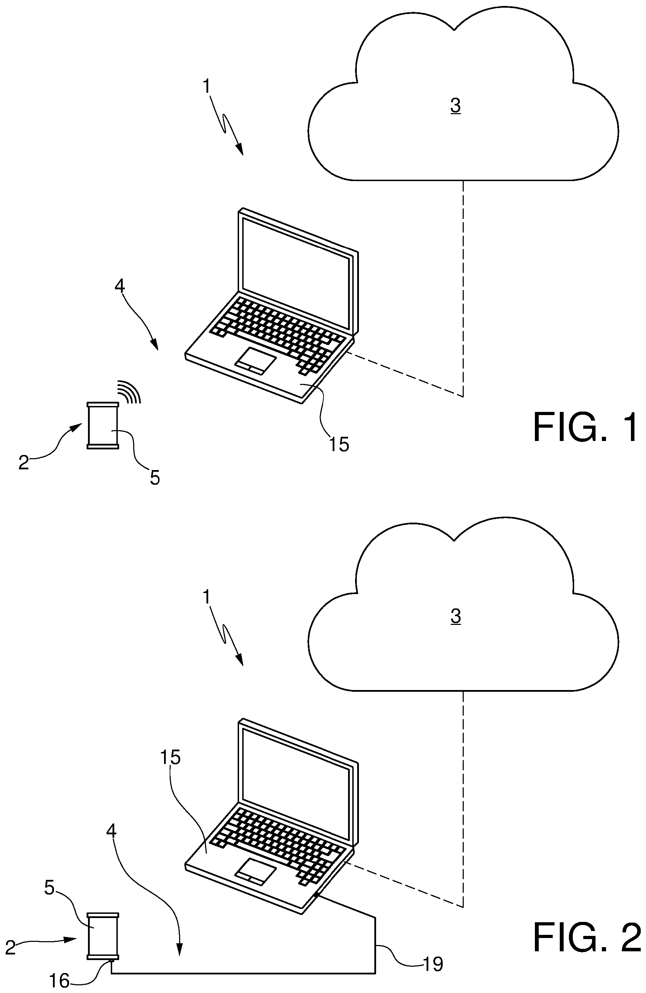

With reference to the annexed figures 1 and 2, the reference number 1 generally indicates an impedentiometric system for the assessment of muscle mass, according to the present invention.

Figures 1 and 2 schematically show the fundamental components of the system 1, which will be described in greater detail hereinafter.

According to one aspect of the invention, the impedentiometric system 1 comprises at least one wearable device 2.

The wearable device 2 comprises an electronic detection part, which acquires the necessary parameters for carrying out the impedance measurement on the subject concerned.

In more detail, the electronic detection part of the wearable device 2 only performs the measurements of the parameters, as well as a first validation of the quality of the measurement performed.

For these reasons, the electronic detection part of the wearable device 2, which is functionally and structurally simple, can be made with very small dimensions and very low weight: it can therefore easily be confined within a wearable device 2. According to another aspect of the invention, the impedentiometric system 1 further comprises at least one cloud platform 3.

The cloud platform 3 is configured so as to receive and process the measurement parameters acquired by the electronic detection part of the wearable device 2.

The reception of the data and the subsequent processing within the context of the cloud platform 3 are carried out according to the rules laid down by the user, for example to produce a medical report or make assessments about the status of the subject/patient.

According to yet another aspect of the invention, the impedentiometric system 1 comprises an interface 4 between the wearable device 2 and the cloud platform 3.

The interface 4 is configured to allow the transmission of impedance measurement data from the wearable device 2 to the cloud platform 3, as better explained hereinafter.

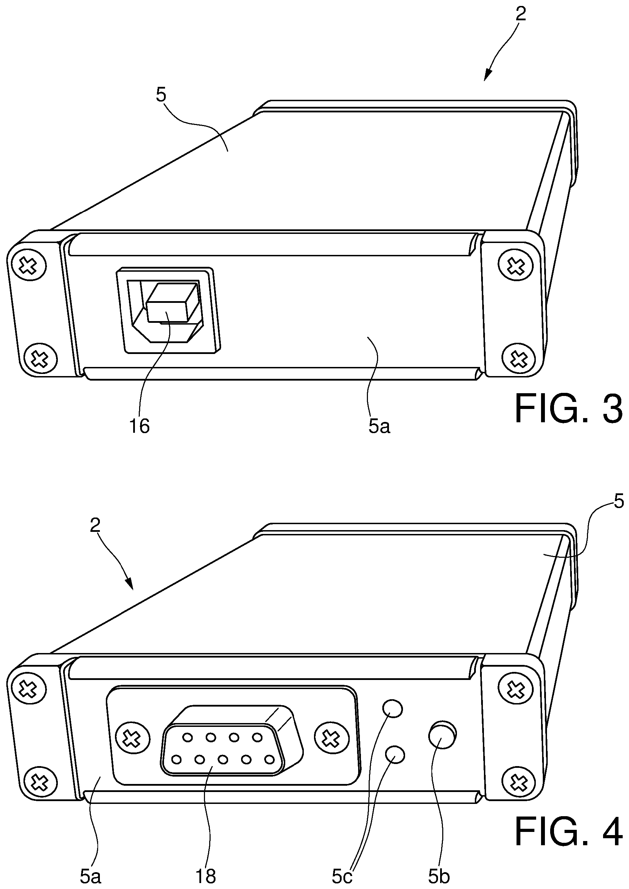

With reference to figures 3 and 4, the wearable device 2 comprises a casing 5.

The casing 5 can be made, for example, in metallic material, or plastic or other suitable material.

The casing 5 can have, for example, a parallelepiped shape, or substantially parallelepiped.

The casing 5 can be made with a flattened shape, so as to be more comfortable when the device 2 is worn by the patient.

The casing 5 can further comprise two opposite bases 5a, which can host connections, a power button 5b, warning lights 5c, or other.

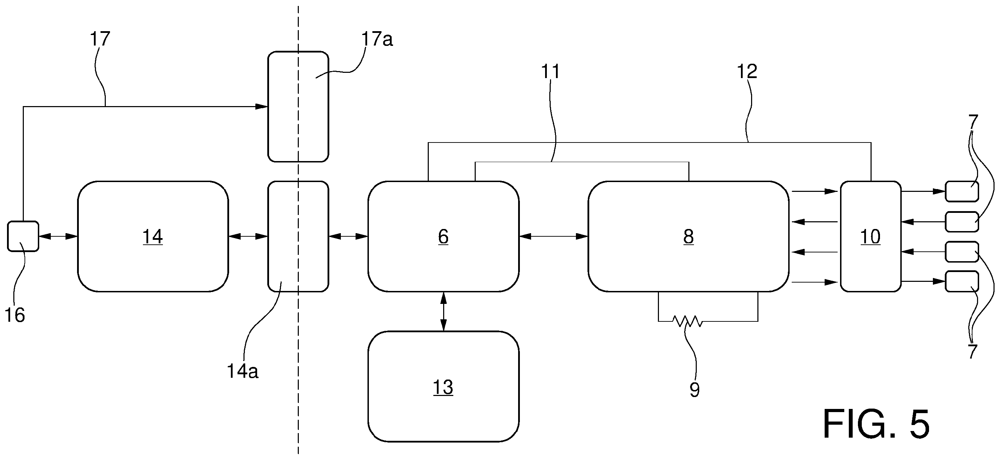

Figure 5 shows a block diagram of the electronic detection part of the wearable device 2, housed inside the casing 5.

The wearable device 2 comprises, in its electronic detection part, a main processor 6, configured for processing the measurement data.

The wearable device 6 further comprises electrodes 7, to be applied at certain positions on the body of the subject/patient (e.g. hands and feet).

The electrodes 7 are connected to the wearable support 2 by means of an isolated cable (not shown in the figures).

The wearable device 2 also comprises an analogue front-end 8, for the generation of the stimulus and the acquisition of the measurements.

The analogue front-end 8 is connected to the main processor 6.

The analogue front-end 8 also comprises a precision resistance 9, for the autocalibration function.

In particular, the analogue front-end 8 generates the measurement signal to apply to the external electrodes 7, and detects the raw impedance value from the internal electrodes.

The raw data is transferred to the main processor 6, for the necessary processing. The analogue front-end 8 also has internal switches required for switching the measurement towards the precision resistance 9, so as to enable the aforesaid autocalibration function at every start-up of the machine.

The wearable device 2 also comprises a measurement bridge 10 for measuring the correct impedance of the electrodes 7: in particular, the bridge 10 allows verifying that all the electrodes 7 are connected, and that the average value of the measurements detected by the same does not exceed a predetermined threshold.

The bridge 10 is connected to the analogue front-end 8.

A clock line 11 is provided, generated by the main processor 6, and connected to the analogue front-end 8, necessary for the measurement system.

A control line 12 is also provided for the management of the electrode measurement bridge 10 measured by the main processor 6.

The wearable device 2 further comprises, in its electronic detection part, components necessary for realising the aforesaid interface 4 for connection to the cloud platform 3.

In particular, according to one aspect of the present invention, the interface 4 comprises at least one Wi-Fi module 13, for the transfer of data in the case of wireless application.

The Wi-Fi module 13 is comprised in the electronic detection part of the wearable device 2.

The Wi-Fi module 13 can also implement a web server/client for autonomously connecting to the cloud platform 3.

Furthermore, according to another aspect of the invention, the interface 4 comprises at least one HID interface processor 14.

The HID interface processor 14 is comprised in the electronic detection part of the wearable device 2.

The interface 4 provided between the wearable device 2 and the cloud platform 3, also comprises a computer 15, or other device which can be connected to the internet (smartphone, tablet, etc.).

The aforesaid HID interface processor 14 is, in particular, configured and programmed to be recognised by the USB system of the computer 15, or other device which can be connected to the internet, such as an HID (Human Interface Device) periphery device; in more detail, it is suitable to emulate the behaviour of a keyboard.

When the wearable device 2 performs the measurement and transfers it to this processor 14, the result will be shown on the page currently active on the computer 15, or other device, and in particular at the point in which it will be the "focus" (for example text box).

This advantageously allows using, for interfacing the apparatus 2 to the cloud platform 4, any device connected to the internet, without any limitation connected to the operating system or the technical specifications of the machine.

Moreover, thanks to this, it is not necessary to install any specific software or driver for interacting with the device 2 on the aforesaid device connected to the internet.

Between the HID interface processor 14 and the main processor 6 a respective galvanic isolation 14a is provided for data transfer, necessary to comply with medical legislation.

The wearable device 2 further comprises a USB socket 16 for connection to the computer 15, or other similar device (for example tablet, smartphone); it is also used to power the device 2 via a respective power supply line 17 (also having the function of battery charging).

The power supply line 17 comprises a respective galvanic isolation 17a, necessary to comply with medical legislation.

The USB socket 16 is connected directly to the HID interface processor 14.

Figure 4 shows a serial port 18 which serves to connect the cables of the electrodes 7 and to recharge the battery of the instrument, so that it is impossible to perform measurements during the battery charging step.

In summary, therefore, the main processor 6 configures the analogue front-end 8 for measurement, extracts the sampled data, generates the clock for the measurement and manages the bridge 10 for measuring the impedance of the electrodes 7.

The main processor 6 has, internally, the algorithms necessary for extracting from the raw data, and affected by noise/interference, the information on the impedance value, mediated and compensated for the non-linearity of the system.

According to another aspect of the invention, the analogue front-end 8 comprises an integrated 800 sps sampler and digital converter.

The analogue front-end 8 is configured so as to provide a high amount of data useful for fine-tuning the measurement and cleaning it from interference and errors caused by patient instability, which can therefore be due to tremors or changes in posture.

The processing of the signal takes place in any case in the cloud platform 3, which allows overcoming the numerous limitations related to the impedance analysis.

Therefore, the wearable device 2 according to the present invention advantageously does not provide for the interposition of band-pass filters to eliminate noise, but is simply configured so as to collect a sampling of 1 second equal to 800 measurements, and determine the minimum, maximum and standard deviation of the sampling carried out, and send it to the computer 15.

According to the invention, therefore, the interface 4 between the wearable device 2 and the cloud platform 3 can be made according to two different configurations. As schematically shown in figure 1, the first configuration is of the Wi-Fi type (via the aforesaid Wi-Fi module 13).

This first configuration allows performing measurements of the patient totally physically independent from the computer 15, thus allowing the detection of the impedance and its variations also following stresses or physical activity.

In this mode, therefore, the wearable device 2 exchanges data wirelessly with the computer 15 which, in turn, thanks to suitable software, sends the results to the cloud platform 3.

The second configuration provides for the use of a USB cable 19, through the HID interface processor 14.

In this mode, the wearable device 2 sends the data to the cloud platform 3 using the HID protocol that is commonly used for interfacing the keyboard to a computer.

The use of this protocol, as mentioned, allows the use of any device (computer 15, tablet, smartphone) to send the measurements to the cloud platform 3 without requiring the installation of any software on the device used.

In practice, the user accesses a website, and after having logged in and inserted the data relative to the patient and to the measurement, sends the data to the cloud platform 3, which will then carry out the reporting.

According to another aspect of the invention, the cloud platform 3 integrates a number of strategies to overcome the previously listed limitations of impedance measurement.

To eliminate the factors related to instrumentation, once the data are sent to the cloud platform 3, the latter performs the following evaluations.

The device 2 is normally supplied with 50 sets of disposable electrodes 7, thus it can perform 50 measurements.

When about 40 measurements have already been performed, or in the event of malfunction of the electrodes 7, the cloud platform 3 is configured so as to send a new set of electrodes 7.

In this way, it is possible to avoid measurement errors caused by old or poorly preserved electrodes 7, or due to the use of the wearable device 2 by the user with non-calibrated electrodes 7.

Furthermore, the cloud platform 3 is configured to perform an evaluation of the consistency of the measurements taken by the wearable device 2.

In particular, the cloud platform 3 is configured so as to check the consistency of the resistance value, measured by the wearable device 2, in relation to the subject/patient's height.

In more detail, the ratio between the resistance and the height of the subject, expressed in metres, must be between 150 and 700 Ω/m to be consistent.

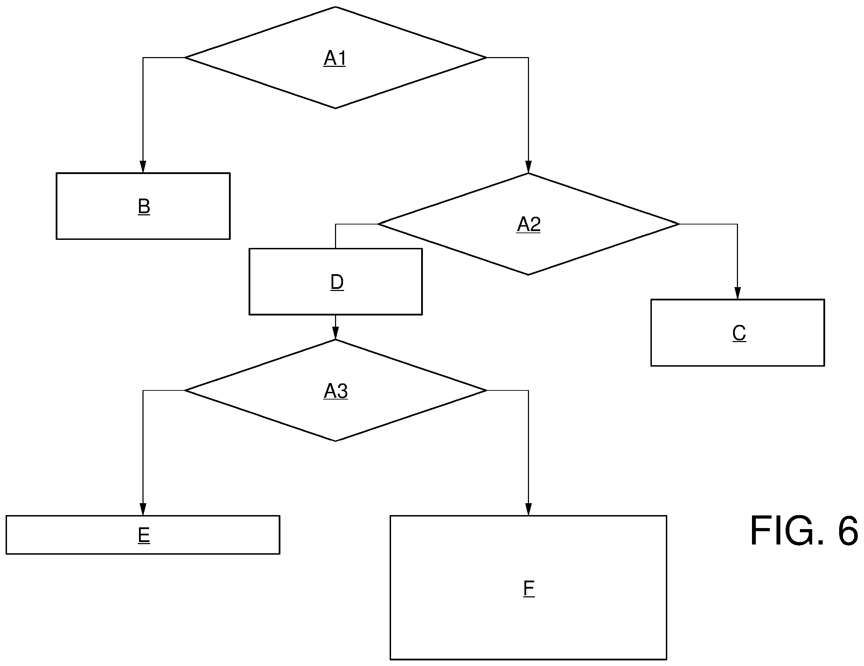

To obviate possible inconsistencies in the measurements performed, the block diagram of figure 6 should be followed.

In a first step of choosing A1, the consistency of the parameter is checked with the above-mentioned range.

In the affirmative case (left arrow) in the executive step B the processing of the data is carried out, by means of the cloud platform 3.

In the negative case (right arrow), a second step of choosing A2 is carried out in which the correctness of the positioning of the electrodes 7 is checked.

In the negative case (right arrow) an executive step C is provided for repositioning the electrodes 7, and the repetition of the measurement.

In the affirmative case (left arrow) an executive step D is provided for carrying out the calibration with a special card without electrodes 7.

The latter executive step D is followed by a further third step of choosing A3 for checking the correctness of the calibration without electrodes.

In the affirmative case (left arrow) an executive step E is provided for replacing the electrodes 7.

In the negative case (right arrow) an executive step F is provided for checking the position of the patient and the cables.

In the case where the data remain inconsistent, then there is a fault with the wearable device 2.

To eliminate the factors relating to the subject to be measured (artefacts linked to posture, the preparation of the skin, the intake of food and beverages, the respiratory dynamics and physical exercise) the cloud platform 3, for each measurement, is configured so as to propose to the user a check list with all the necessary measures to perform a good measurement (how to position the patient, how to cleanse the skin and how to position the electrodes 7).

When for any reason it is not possible to perform a measurement in optimal conditions (due to the cell body of the patient or for any disabilities), the cloud platform 3 is configured so as to record all the variations (posture, breaths per minute, inclination of the bust); in this way the variability linked to the operator is reduced and very reproducible measurements are obtained by always positioning the subject in the same way in the subsequent measurements.

Furthermore, the cloud platform 3 is configured so as to record the last intake of food and beverages and the last session of physical exercise.

As for artefacts related to the presence of tremors (especially, therefore, in relation to elderly subjects) it can be seen that a large part of the literature on the measurement of muscle mass uses 50 Hz as the base frequency.

This generates technical problems, since the muscle tremors in the elderly are comprised between 4 Hz and 12 Hz (the lowest frequencies are typical of Parkinson's Disease, while the higher ones are typical of essential tremors), then they are found in a range of frequencies close to that which is measured.

The direct application of a band-pass filter is not a viable solution because it would alter the measurement of the phase angle.

It should also be noted that the effect of the tremor on the impedance measurement depends both on the type of tremor (e.g. tremor at rest or during movement) and by the amount of muscle affected by the phenomenon.

In fact, it can be assumed that a tremor that affects small muscle groups is substantially irrelevant on the impedance measurement, which is carried out on the whole body; this is not true for tremors that affect the large muscle groups.

Moreover, the respiratory frequency (around 3 Hz) can also alter the impedance measurement, and this is one of the reasons for which in the current state, these measurements are taken with the patient at rest.

According to the present invention, to eliminate artefacts related to the presence of tremors, the cloud platform 3 is configured so as to calculate a change coefficient CV, which is the ratio of the standard deviation to the average of the measurements obtained.

If this ratio is greater than 5%, the cloud platform 3 is configured to perform a Fourier analysis on packets of contiguous data, with which to identify specific noise frequencies and implement special software filters for the elimination of the same, making the system much more tolerant to measurement interference.

In the case where the subject/patient has diseases that can alter the impedance measurement, the cloud platform 3 is configured so as to guide the user through the performance of some ultrasound scans of the subcutaneous layer of the tibial region in the medial portion, and of the medial portion of the calf of the subject.

In this way, in subsequent checks it can be verified if the impedance changes are due to a different distribution of liquids or to an actual change of the muscle mass. Another important characteristic of the cloud platform 3 of the system 1 according to the present invention consists in the fact that it is configured in such a way as to allow for the updating of the mathematical models needed for reporting, without assistance actions on single wearable devices 2.

This solves one of the fundamental problems which limits the diffusion of impedance analysis: the development of new models, in fact, is not rapidly adopted by the instruments, since there may be difficulties in updating the software.

These difficulties make it hard to compare the results obtained with different appliances, perhaps using different mathematical instruments to obtain the reporting.

Moreover, in the case of an update of the mathematical instruments, all the previous measurements of the patient can also be calculated, so as to be able to verify the evolution of the patient him/herself in time with the always-updated models.

It has thus been seen how the invention achieves the intended purposes.

First of all, the electronic design of the wearable device 2 and the cloud platform 3 allows the execution of numerous serial measurements, thus allowing the elimination of any damaging effects due to tremors or movement disorders, which are extremely frequent in elderly subjects.

Moreover, the resolution of the problems related to the reproducibility of the impedance measurement could lead to a greater diffusion of the same in the assessment of muscle disorders of the elderly patient, constituting an economic advantage compared with other currently used methods, and could potentially significantly contribute to the prevention of disability linked to ageing.

The proposed solution also allows overcoming the limitation represented by the impossibility of measuring impedances during movement: this might allow obtaining useful parameters for studying the response to physical exercise, and in particular to rehabilitation.

This result is mainly obtained, as mentioned, thanks to the fact that the device 2, which is concerned only with the acquisition of the measurements, has an extremely small size and low weight, and therefore can easily be worn to perform the measurements during movement.

It should be underlined that the impedentiometric system according to the present invention is distinguished by the extremely low construction and management costs.

Moreover, the use of the system is extremely simple and practical, also for not highly specialized personnel.

The present invention has been described according to preferred embodiments, but the invention is defined by the appended claims.

## Claims

1. An impedentiometric system (1) for the assessment of muscle mass, comprising
at least one wearable device (2), provided with external electrodes (7) to be applied at certain positions on the patient's body, and an electronic detection part suitable only for acquiring the parameters necessary for carrying out the impedance measurement on the subject concerned,
at least one cloud platform (3), configured so as to receive and process the measurement parameters acquired by said electronic detection part of said wearable device (2), and
at least one interface (4) comprising a computer (15) or another device suitable for connection to the Internet configured to allow the transmission of impedance measurement data from said wearable device (2) to said cloud platform (3),
said cloud platform (3) being configured so as to check the consistency of the resistance value, measured by said wearable device (2), in relation to the subject/patient's height,
and wherein said interface (4) comprises at least one Human Interface Device, HID, interface processor (14), included in said electronic detection part of said wearable device (2), configured to receive the impedance measurement data and to be recognised by the USB system of said computer (15), or other device suitable for connection to the Internet, such as a HID peripheral device,
and a USB socket (16) so that said HID interface processor (14) can be connected to said computer (15) by means of a USB cable (19), said HID interface processor being configured for emulating the behaviour of a keyboard.

2. An impedentiometric system (1) according to claim 1, wherein said interface (4) comprises at least one Wi-Fi module (13), included in said electronic part for detecting said wearable device (2), for the transfer of data in wireless mode from said wearable device (2) to said cloud platform (3).

3. An impedentiometric system (1) according to any one of the preceding claims, wherein said electronic detection part of said wearable device (2) comprises a main processor (6) and an analogue front-end (8), connected to said main processor (6), designed to generate the measurement signal to be applied to said external electrodes (7).

4. An impedentiometric system (1) according to claim 3, wherein said analogue front-end (8) comprises an integrated 800 sps sampler and digital converter.

5. An impedentiometric system (1) according to claim 4, wherein said analogue front-end (8) is configured so as to provide a high amount of data useful for fine-tuning the measurement and cleaning it from interference and errors caused by patient instability, which therefore may be due to tremors or changes in posture.

6. An impedentiometric system (1) according to claim 4 or 5, wherein said wearable device (2) is configured so as to determine the minimum, the maximum and the standard deviation of the sampling carried out, and to send it to said cloud platform (3) through said interface (4).

7. An impedentiometric system (1) according to claim 1, wherein said relationship between resistance and height of the subject/patient must be between 150 and 700 Ω/m to be considered consistent.

8. An impedentiometric system (1) according to one of the preceding claims, wherein said cloud platform (3), for each measurement, is configured in such a way as to propose to the user a check list containing the necessary measures to perform a good measurement on the subject/patient.

9. An impedentiometric system (1) according to one of the preceding claims, wherein, to eliminate artefacts related to the presence of tremors in the subject/patient, said cloud platform (3) is configured so as to calculate a change coefficient which is the ratio of the standard deviation to the average of the measurements obtained.

10. An impedentiometric system (1) according to one of the preceding claims, wherein, in the event that the subject/patient suffers from diseases capable of altering the impedance measurement, said cloud platform (3) is configured in such a way as to guide the user through the performance of some ultrasound scans of the subcutaneous layer of the tibial region in the medial portion, and of the medial portion of the calf of the subject, so as to check, in subsequent examinations, whether the impedance changes are due to a different distribution of liquids or to an actual change of the muscle mass.

## Patentansprüche

1. Ein impedentiometrisches System (1) zur Bestimmung der Muskelmasse, bestehend
aus mindestens einem tragbaren Gerät (2), das mit externen Elektroden (7) zur Anbringung an bestimmten Körperstellen des Patienten ausgestattet ist, und einem elektronischen Erfassungsteil, das ausschließlich zur Erfassung der für die Impedanzmessung am betreffenden Probanden erforderlichen Parameter geeignet ist,
mindestens einer Cloud-Plattform (3), die die vom elektronischen Erfassungsteil des tragbaren Geräts (2) erfassten Messparameter empfängt und verarbeitet, und
mindestens einer Schnittstelle (4), die einen Computer (15) oder ein anderes internetfähiges Gerät umfasst und die Übertragung von Impedanzmessdaten vom tragbaren Gerät (2) an die Cloud-Plattform (3) ermöglicht,
die besagte Cloud-Plattform (3) ist so konfiguriert, dass sie die Konsistenz des vom tragbaren Gerät (2) gemessenen Widerstandswerts in Bezug auf die Körpergröße des Probanden/Patienten überprüft,
und wobei die Schnittstelle (4) umfasst mindestens einen Human Interface Device (HID)-Schnittstellenprozessor (14), der im elektronischen Erfassungsteil des tragbaren Geräts (2) integriert ist und konfiguriert ist, um die Impedanzmessdaten zu empfangen und um vom USB-System des Computers (15), oder einem anderen internetfähigen Gerät, wie z. B. einem HID-Peripheriegerät, erkannt zu werden,
und eine USB-Buchse (16), sodass der HID-Schnittstellenprozessor (14) über ein USB-Kabel (19) an den Computer (15) angeschlossen werden kann, der besagte HID-Schnittstellenprozessor ist so konfiguriert, dass er das Verhalten einer Tastatur emuliert.

2. Ein impedentiometrisches System (1) gemäß Anspruch 1, wobei die Schnittstelle (4) mindestens ein Wi-Fi-Modul (13) umfasst, das im elektronischen Erfassungsteil des tragbaren Geräts (2) enthalten ist, für die drahtlose Übertragung von Daten vom tragbaren Gerät (2) zur Cloud-Plattform (3).

3. Ein impedenzometrisches System (1) gemäß einem der vorhergehenden Ansprüche, wobei der elektronische Erfassungsteil des tragbaren Geräts (2) einen Hauptprozessor (6) und ein analoges Front-End (8) umfasst, das mit dem Hauptprozessor (6) verbunden ist und dazu dient, das Messsignal zu erzeugen, das an die externen Elektroden (7) angelegt werden soll.

4. Ein impedenzometrisches System (1) nach Anspruch 3, wobei das analoge Front-End (8) einen integrierten 800-sps-Abtastwertgeber und Digitalkonverter umfasst.

5. Ein impedenzometrisches System (1) gemäß Anspruch 4, wobei das analoge Front-End (8) so konfiguriert ist, dass es eine große Menge an Daten liefert, die für die Feinabstimmung der Messung und die Bereinigung von Störungen und Fehlern nützlich sind, die durch Instabilität des Patienten verursacht werden, die daher auf Zittern oder Haltungsänderungen zurückzuführen sein können.

6. Ein impedenzometrisches System (1) gemäß Anspruch 4 oder 5, wobei das tragbare Gerät (2) so konfiguriert ist, dass es die minimale, die maximale und die Standardabweichung der durchgeführten Probenahme bestimmt und diese über die Schnittstelle (4) an die Cloud-Plattform (3) sendet.

7. Ein impedenzometrisches System (1) nach Anspruch 1, wobei die Beziehung zwischen Widerstand und Größe des Subjekts/Patienten zwischen 150 und 700 Ω/m liegen muss, um als konsistent zu gelten.

8. Ein impedentiometrisches System (1) gemäß einem der vorhergehenden Ansprüche, wobei die Cloud-Plattform (3) für jede Messung so konfiguriert ist, dass sie dem Benutzer eine Checkliste mit den notwendigen Maßnahmen vorschlägt, um eine gute Messung am Probanden/Patienten durchzuführen.

9. Ein impedentiometrisches System (1) gemäß einem der vorhergehenden Ansprüche, wobei die Cloud-Plattform (3), zum Eliminieren von Artefakten, die mit dem Vorhandensein von Tremor beim Subjekt/Patienten in Zusammenhang stehen, so konfiguriert ist, dass sie einen Änderungskoeffizienten berechnet, der das Verhältnis der Standardabweichung zum Durchschnitt der erhaltenen Messungen darstellt.

10. Ein impedentiometrisches System (1) gemäß einem der vorhergehenden Ansprüche, wobei für den Fall, dass das Subjekt/der Patient an Krankheiten leidet, die die Impedanzmessung verändern können, die Cloud-Plattform (3) so konfiguriert ist, dass sie den Benutzer durch die Durchführung einiger Ultraschalluntersuchungen der subkutanen Schicht der Tibiaregion im medialen Bereich und des medialen Bereichs der Wade des Subjekts führt, um in nachfolgenden Untersuchungen zu überprüfen, ob die Impedanzänderungen auf eine unterschiedliche Flüssigkeitsverteilung oder auf eine tatsächliche Veränderung der Muskelmasse zurückzuführen sind.

## Revendications

1. Système impédentiométrique (1) pour l'évaluation de la masse musculaire, comprenant
au moins un dispositif portable (2) muni d'électrodes externes (7) à appliquer à certains endroits du corps du patient, et une partie de détection électronique destinée uniquement à acquérir les paramètres nécessaires à la mesure d'impédance du sujet concerné;
au moins une plateforme cloud (3) configurée pour recevoir et traiter les paramètres de mesure acquis par ladite partie de détection électronique du dispositif portable (2); et
au moins une interface (4) comprenant un ordinateur (15) ou un autre appareil adapté à la connexion à Internet, configuré pour permettre la transmission des données de mesure d'impédance du dispositif portable (2) à ladite plateforme cloud (3),
ladite plateforme cloud (3) étant configurée pour vérifier la cohérence de la valeur de résistance mesurée par le dispositif portable (2) par rapport à la taille du sujet/patient,
et dans lequel ladite interface (4) comprend au moins un Human Interface Device, HID, processeur d'interface (14), intégré à la partie de détection électronique du dispositif portable (2), configuré pour recevoir les données de mesure d'impédance et être reconnu par le système USB dudit ordinateur (15), ou tout autre appareil connecté à Internet, tel qu'un périphérique HID,
et une prise USB (16) permettant de connecter ledit processeur d'interface HID (14) audit ordinateur (15) au moyen d'un câble USB (19), ledit processeur d'interface HID étant configuré pour émuler le comportement d'un clavier.

2. Système impédentiométrique (1) selon la revendication 1, dans lequel ladite interface (4) comprend au moins un module Wi-Fi (13), inclus dans ladite partie de détection électronique dudit dispositif portable (2), pour le transfert de données en mode sans fil dudit dispositif portable (2) vers ladite plateforme cloud (3).

3. Système impédentiométrique (1) selon l'une quelconque des revendications précédentes, dans lequel ladite partie de détection électronique dudit dispositif portable (2) comprend un processeur principal (6) et un frontal analogique (8), connecté audit processeur principal (6), conçu pour générer le signal de mesure à appliquer auxdites électrodes externes (7).

4. Système impédentiométrique (1) selon la revendication 3, dans lequel ledit frontal analogique (8) comprend un échantillonneur 800 sps intégré et un convertisseur numérique.

5. Système impédentiométrique (1) selon la revendication 4, dans lequel ledit frontal analogique (8) est configuré de manière à fournir une grande quantité de données utiles pour affiner la mesure et la nettoyer des interférences et des erreurs causées par l'instabilité du patient, qui peut donc être due à des tremblements ou à des changements de posture.

6. Système impédentiométrique (1) selon la revendication 4 ou 5, dans lequel ledit dispositif portable (2) est configuré de manière à déterminer le minimum, le maximum et l'écart type de l'échantillonnage effectué, et à l'envoyer à ladite plateforme cloud (3) via ladite interface (4).

7. Système impédentiométrique (1) selon la revendication 1, dans lequel ladite relation entre la résistance et la taille du sujet/patient doit être comprise entre 150 et 700 Q/m pour être considérée comme cohérente.

8. Système impédentiométrique (1) selon l'une des revendications précédentes, dans lequel ladite plateforme cloud (3), pour chaque mesure, est configurée de manière à proposer à l'utilisateur une liste de contrôle contenant les mesures nécessaires pour réaliser une bonne mesure sur le sujet/patient.

9. Système impédentiométrique (1) selon l'une des revendications précédentes, dans lequel, pour éliminer les artefacts liés à la présence de tremblements chez le sujet/patient, ladite plateforme cloud (3) est configurée de manière à calculer un coefficient de variation qui est le rapport de l'écart type à la moyenne des mesures obtenues.

10. Système impédentiométrique (1) selon l'une des revendications précédentes, dans lequel, dans le cas où le sujet/patient souffre de maladies susceptibles d'altérer la mesure d'impédance, ladite plateforme cloud (3) est configurée de manière à guider l'utilisateur à travers la réalisation de quelques échographies de la couche sous-cutanée de la région tibiale dans la partie médiale, et de la partie médiale du mollet du sujet, de manière à vérifier, lors d'examens ultérieurs, si les changements d'impédance sont dus à une distribution différente des liquides ou à un changement réel de la masse musculaire.
